# EUROPEAN PATENT SPECIFICATION

(11) **EP 0 597 331 B1**
(45) Date of publication and mention of the grant of the patent: **19.11.1997**
(21) Application number: 93117507.9
(22) Date of filing: 16.12.1988
(51) Int. Cl.: A61F 5/44, A61F 13/15, A41B 13/04

(54) **A garment for absorbing human discharge**
Kleidung zum Absorbieren menschlicher Ausscheidung
Vêtement pour l'absorption de décharge humaine

(30) Priority: 16.12.1987 US 133674; 16.12.1987 US 133758
(43) Date of publication of application: 18.05.1994
(62) Divisional of application: 88121163.5
(73) Proprietor: KIMBERLY-CLARK CORPORATION, Neenah, Wisconsin 54956-0349 (US)
(72) Inventor: Van Gompel, Paul Theodore, Hortonville, WI 54944 (US); Surprise, Jody Dorothy, Neenah, WI 54956 (US); Schleinz, Robert Joseph, Appleton, WI 54915 (US); Boland, Leona Gertrude, Neenah, WI 54956 (US); Zehner, Georgia Lynn, Larsen WI 54957 (US)
(74) Representative: Diehl, Hermann, Dr. Dipl.-Phys.

(56) References cited:
- GB-A- 1 520 740
- US-A- 2 141 105
- US-A- 4 437 860
- US-A- 4 486 192
- US-A- 4 655 760

## Description

This invention pertains to an absorbent garment, and more particularly to an absorbent garment for use as a child's training pant, adult incontinence garment, baby diaper and the like.

Currently, disposable absorbent garments find widespread use for infant care and adult incontinence care, and have generally replaced the use of reusable cloth absorbent garments, such as cloth diapers. The typical disposable absorbent garment is a three-layered composite structure comprising a liquid-permeable bodyside liner, a liquid-impermeable outer cover and an absorbent batt disposed between the bodyside liner and the outer cover. Materials now in general use for the three principal elements of a disposable absorbent garment include various types of nonwoven fabrics for the bodyside liner, a thin thermoplastic film for the outer cover and cellulosic fluff for the absorbent batt.

As one type of a disposable absorbent garment, diapers presently on the market are flat open-sided garments that are intended to be fit about an infant while lying down. A diaper is meant for use when the child is young and dependent upon a parent for fitting the diaper on the child.

The popularity of disposable diapers has led us to believe there is a demand for a disposable training pant that can be used when a child grows out of a diaper. Diapers are typically used with infants up to about 15 months old. When a child reaches an age in the range of about 15 to 30 months, a parent generally desires to start toilet training so the child can become independent of the parent. The training pant is intended for use when the child has reached an age at which he or she is ready to graduate to an underpant type of garment as a replacement for disposable diapers previously used. Thus, a suitable training pant must be a garment having closed sides so that a child can raise and lower the garment as necessary without requiring the aid of a parent. At the same time, a training pant must provide features of liquid and solid absorbency and prevent leakage of the waste fluids.

Cloth training pants, although widely used, have disadvantages. Current cloth training pants have very little absorbency and often must be used with exterior rubber or plastic pants. When a child wets a cloth training pant, most often all of the child's clothes must be changed. Further, if a child has a bowel movement, it is difficult to remove a cloth pant without making a mess, and the pant must be soaked and bleached. All of these factors can make the toilet training process frustrating for both child and parent.

Moreover, it is believed that the psychology of the toilet training stage is such that the child should perceive he or she is graduating to a garment that is different than a disposable diaper. It is evident that there is a need for a child's disposable training pant that meets the above requirements regarding fit, leakage, and psychology of toilet training.

One type of disposable absorbent article is described in US-A-4,205,679. This disposable absorbent article is a panty adapted for use in training infants or for use by incontinent adults or children. It is constructed from a non-woven stretchable fabric and comprises a front portion, a rear portion, a crotch portion, a self-fitting waist portion and self-fitting leg apertures. The fit in the leg area is enhanced by suitably depositing beads of hot melt elastics.

As another type of disposable absorbent article, some of the currently-used incontinence products for adults and older children have been found unsatisfactory due to their bulkiness and ineffectiveness. Many of these garments are formed by folding flat sheets into a diaper-like structure that is bulky, particularly in the crotch portion. This type of garment further has a tendency to became dislodged during activity. Clearly, for the active person, these diaper-type garments are not desirable since they are bulky and interfere with the movements of the individual and the wearing of ordinary clothes. Furthermore, the large amounts of material utilized requires these adult diaper-type garments to be relatively expensive. An example of the above mentioned garments is the sanitary garment described in document GB-A-1 520 740.

It is, therefore, an object of this invention to provide an improved disposable pant-like garment for absorbing human discharge. This object is solved by the disposable pant-like garment as disclosed in independent claim 1. Further advantageous features of the garment are evident from the dependent claims.

In one form of the invention, there is provided a disposable pant-like garment for absorbing human discharge, comprising an absorbent assembly including an outer cover and an absorbent medium. The absorbent assembly further includes generally opposite side edges and generally opposite end edges. Stretchable side panels are joined to the side edges of the absorbent assembly forming waist and leg openings. A gathering means is joined at least along a portion of each leg opening for gathering that portion about the leg of a wearer.

The above mentioned and other features and objects of this invention and the manner of attaining them will become more apparent, and the invention itself will be better understood by reference to the following description of the invention taken in conjunction with the accompanying drawings, wherein:
Figure 1 is a perspective view of one embodiment of a garment or pant as it would appear on a wearer indicated in dashed lines;
Figure 2 is a perspective view of another embodiment on a wearer indicated in dashed lines;
Figure 3 is a front elevational view of the embodiment in Figure 1;
Figure 4 is a front elevational view of the embodiment in Figure 2;
Figure 5 is a top plan view of the embodiment of Figure 1 in a flat condition with leg cut-outs before the seams are joined;
Figure 5A is a sectional view of Figure 5 taken along line 5A-5A;
Figure 5B is a modification of the view in Figure 5A;
Figure 5C is a modification of the view in Figure 5B;
Figure 6 is similar to Figure 5 and has the nonelastic intermediate panel and waist elastics removed;
Figure 7 is a top plan view of the embodiment of Figure 2 in a flat condition before the seams are joined;
Figure 8 is a side plan view of the embodiment of Figure 7 in a partially folded form;
Figure 9 is a fragmentary, side cross-sectional view of a stretch-bonded laminate in the stretched condition;
Figure 10 is a fragmentary, side cross-sectional view of the stretch-bonded laminate of Figure 9 in a relaxed condition;
Figure 11 is a sectional view of Figure 5 taken along line 11-11 and viewed in the direction of the arrows;
Figure 12 illustrates a modification of the view of Figure 11;
Figure 13 illustrates a modification of the view of Figure 11;
Figure 14 is a sectional view of Figure 5 taken along line 14-14 and viewed in the direction of the arrows;
Figure 15 illustrates a modification of the view of Figure 14;
Figure 16 illustrates a modification of the view of Figure 14;
Figure 17 is a graph of percent elongation of a side panel versus fit range of the waist in inches*;
Figure 18 is a graph of the side panel width in inches* versus the fit range of the waist in inches *;
Figure 19 is a graph of percent elongation versus the load in grams for a one inch*wide strip of side elastic;
Figure 20 is a schematic of one apparatus for producing one embodiment of a garment or pant; and
Figure 21 illustrates yet another embodiment of a garment or pant.

* 1 inch = 2.54 cm

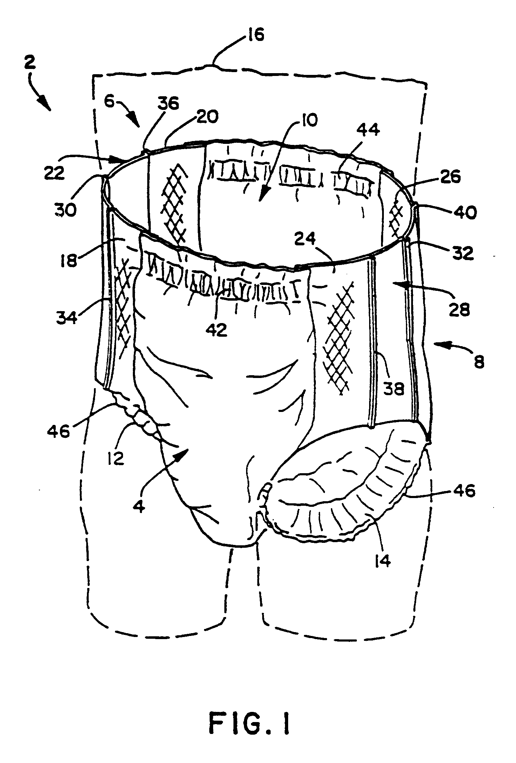

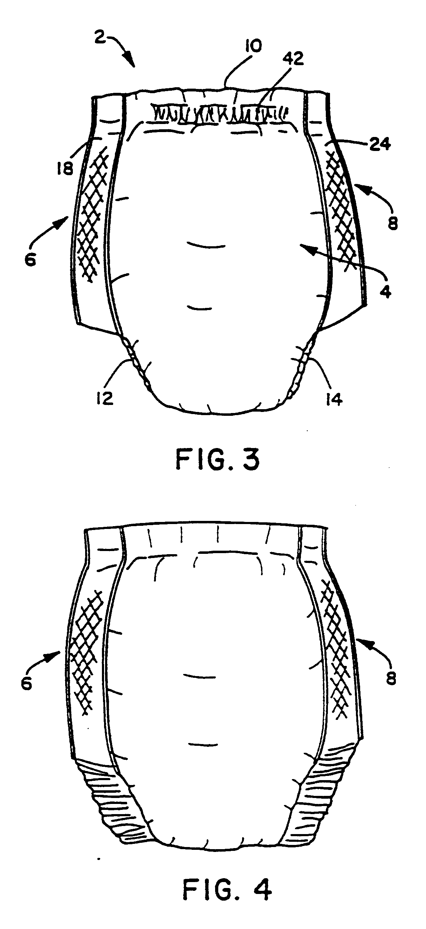

Referring to Figures 1 and 3, there is illustrated one embodiment designated absorbent garment 2. Garment 2 generally comprises waste containment section 4 and two side panels 6, 8 defining a waist opening 10 and a pair of leg openings 12, 14. The total surface area of both side panels 6, 8 comprises about 20% to about 80% of the total surface area of garment 2, preferably about 25% to about 50%, and more preferably about 35% to about 45%. Figure 1 illustrates absorbent garment 2 fitted on a wearer's torso portion 16 in dashed lines. Side panel 6 includes stretchable side member 18 and stretchable side member 20 connecting intermediate member 22 which is made of a nonstretchable material. Similarly, side panel 8 includes stretchable side member 24 and stretchable side member 26 connecting intermediate member 28 which is made of a nonstretchable material. As illustrated in Figure 1, both intermediate members 22, 28 are made of two halves joined by respective seams 30, 32. Similarly, seam 34 joins stretchable side member 18 to intermediate member 22, seam 36 joins stretchable side member 20 to intermediate member 22, seam 38 joins stretchable side member 24 to intermediate member 28 and seam 40 joins stretchable side member 26 to intermediate member 28.

Hereafter, the terms "elasticity", "stretchability", and "elongation" will be interchangeably used to describe the properties of various materials. The meaning of these three words is intended to be the same, and that is that the material can be stretched and, upon relaxing, will tend to resume its original shape.

Garment 2 also includes front waist elastic member 42 and rear waist elastic member 44 for providing additional elasticity along waist opening 10. Leg elastics 46 are provided with waste containment section 4 between side panels 6, 8.

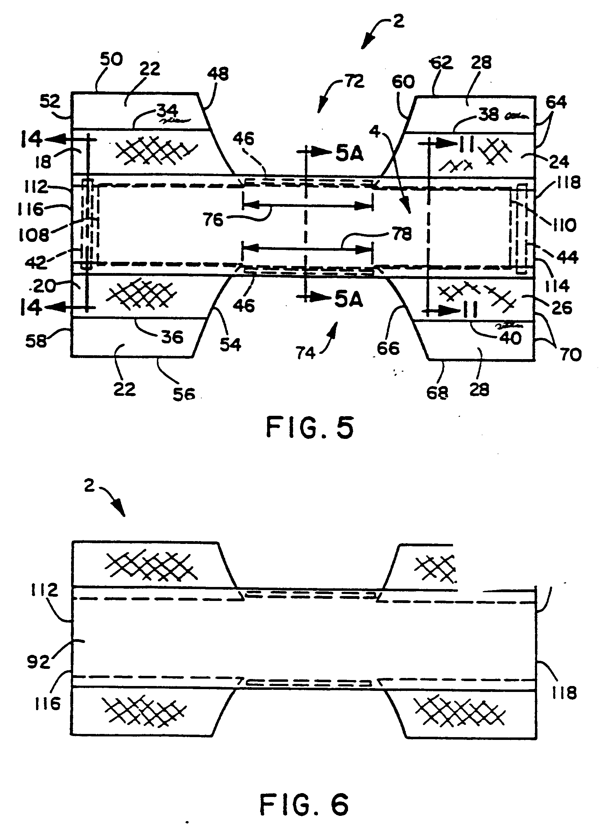

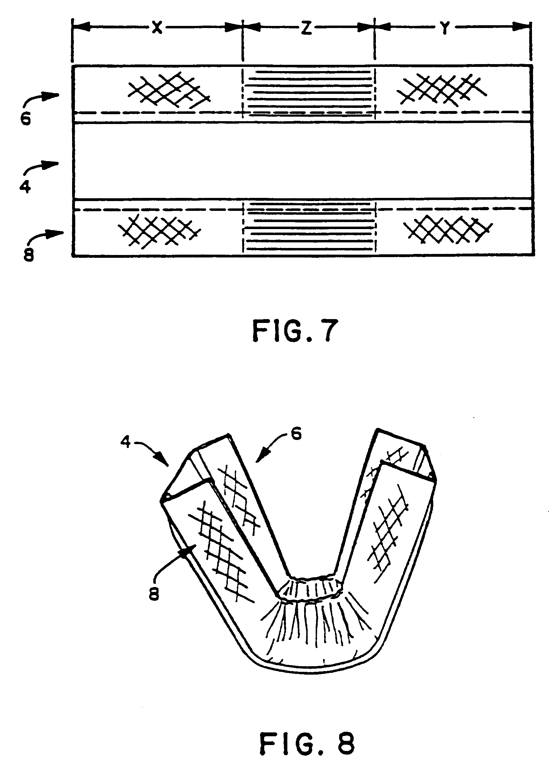

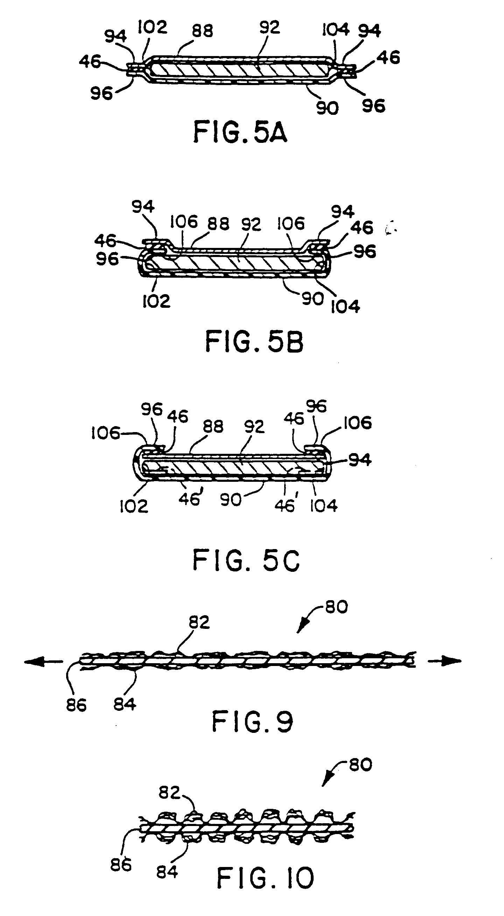

Referring now to Figure 5, absorbent garment 2 is illustrated in a two-dimensional or planar configuration it assumes during the manufacturing process. Stretchable side member 18 is joined to waste containment section 4 and to a portion of intermediate member 22 at seam 34, and together form inner edge 48, side edge 50 and end edge 52. Similarly, stretchable side member 20 and its portion of intermediate member 22, which are joined at seam 36, form inner edge 54, side edge 56 and end edge 58. Although intermediate member 22 is illustrated in two halves in Figure 5, it may be one integral member without a seam in the middle.

Stretchable side member 24 and its portion of intermediate member 28, which are joined at seam 38, form inner edge 60, side edge 62 and end edge 64. Stretchable side member 26 and its portion of intermediate member 28, which are joined at seam 40, form inner edge 66, side edge 68 and end edge 70. Again, intermediate member 28 can be of a form or geometry permitting it to be attached as one complete part to either stretchable side member 24 or 26, as opposed to being attached in two portions as illustrated in Figure 5. Generally, the length of a side edge 50, 56, 62 or 68 is a function of the total garment length, e.g., a desired length of a side edge 50, 56, 62 or 68 is about 5% to about 50% of total garment length. A preferred length is about 15% to about 40% total garment length and a more preferred length is about 30% to about 40% total garment length. Total garment length is measured generally between end edge 52 and end edge 64, or between end edge 58 and end edge 70. Total garment length will generally be between about 12 inches to about 30 inches. Total garment width, as measured between edge 50 and edge 56 or between edge 62 and edge 68, is generally between about 6 inches to about 30 inches.

As described, leg cutout 72 is formed by inner edges 48, 60 and intermediate portion 76 of waste containment section 4 along leg elastic 46. Similarly, leg cutout 74 is formed by inner edges 54, 66 and intermediate portion 78 of waste containment section 4 along the other leg elastic 46. Although illustrated in Figure 5 as being symmetrical about both its longitudinal and transverse axes, absorbent garment 2 may be varied in design or configuration by, for example, changing the dimension of intermediate portions 76, 78 or moving intermediate portions 76, 78 toward the front waist elastic member 42 or toward the waist elastic member 44. Any such redesign or reconfiguration will naturally be accompanied by a change in design or configuration of stretchable side members 18, 20, 24, 26 and intermediate members 22, 28. The design or configuration of absorbent garment 2 can also be varied by changing the curvatures of inner edges 48, 54, 60 and 66, such as by changing their radius of curvature.

Stretchable side members 18, 20, 24, 26 can be made of a single layer of a woven or nonwoven elastic or stretchable material, such as block copolymers of polystyrene, polyisoprene or polybutadiene, copolymers of ethylene, natural rubbers, urethanes, Kratons, and coextrusions/blends of the afore-mentioned. Other examples of suitable elastomeric materials include copolymers of ethylene, ethylene-vinyl acetate, ethylene-ethyl acetate, ethylene-acrylic acid, and ethylene-methyl acrylate and various percent blends of the copolymers of ethylene with polypropylene. Coextruded composites of ethylene-vinyl acetate, ethylene-ethyl acetate, ethylene-acrylic acid, ethylene-methyl acrylate, and polypropylene at various percents or mil thicknesses can also be used as the elastic material. Also, elastomeric staple integrated composites where staple fibers such as polypropylene, polyester, cotton or any other suitable staple fiber are integrated into an elastomeric meltblown web. Stretchable side members 18, 20, 24, 26 can also be a film of elastomeric material.

The above elastomeric materials may be formed by any suitable processes, such as film extrusion, spunbond process, meltblown process or the like.

Stretchable side members 18, 20, 24, 26 can also be a stretch-bonded laminate that may have elasticity in all directions, and may be breathable, i.e., is pervious to vapors, but impervious to liquids. Figures 9 and 10 illustrate a stretch-bonded laminate 80 in the stretched and relaxed conditions, respectively. Stretch-bonded laminate 80 generally comprises an outer layer 82, an inner bodyside layer 84 and an elastic layer 86 disposed between layers 82, 84. Although layers 82, 84 are described as outer and inner, respectively, they can be made of the same materials and thus be interchangeable.

Layers 82, 84 can be made of any woven or nonwoven material, and are preferably made of a nonwoven fibrous material. Examples of nonwoven fibrous material include variously bonded polyolefin fibers such as thermally-bonded polypropylene, polyethylene, polyester; spunbonded polypropylene, spunbonded polyethylene or blends thereof; meltblown polypropylene, meltblown polyethylene or blends thereof; bonded carded webs of synthetic or natural fibers or blends thereof; extruded films of thermoplastic materials; and the like. Naturally, copolymers of polyolefin or other material fibers may also be utilized.

Elastic or stretchable layer 86 is preferably a meltblown or film of block or graft copolymers such as butadiene, isoprene, styrene, ethylene-methyl acrylate, ethylene-vinyl acetate, ethylene-ethyl acrylate or blends thereof. One preferred elastomeric is a block copolymer of styrene-ethylbutadiene-styrene. Other types of materials of which elastic layer 86 can be made are a meltblown or film of block or graft copolymers such as butadiene, isoprene, styrene, ethylene-methyl acrylate, ethylene-vinyl acetate, ethylene-ethyl acrylate or blends thereof. One preferred elastomeric is a block copolymer of styrene-ethylbutadiene-styrene. Other types of materials of which elastic layer or stretchable layer 86 can be made are the Kraton G series from The Shell Chemical Company such as Kraton G-1650, Kraton G-1652, Kraton GX-1657 and preferably Kraton G-2740X. Also, the Kraton D series can be used, as well as polyester elastomeric materials, polyurethane elastomeric materials and polyamide elastomeric materials. It should be pointed out that the stretchable or elastomeric materials of which side panels 6, 8 are made can also be used for layer 86, and the just-described stretchable or elastomeric materials of which layer 86 can be made may also be used to make side panels 6, 8.

Typically, a stretch-bonded laminate is made by stretching the elastic or stretchable layer to a selected elongation; placing a non-stretched layer or layers on the stretched elastic layer; bonding the layers together and allowing the layers to relax so that the elastic layer gathers the other layer or layers.

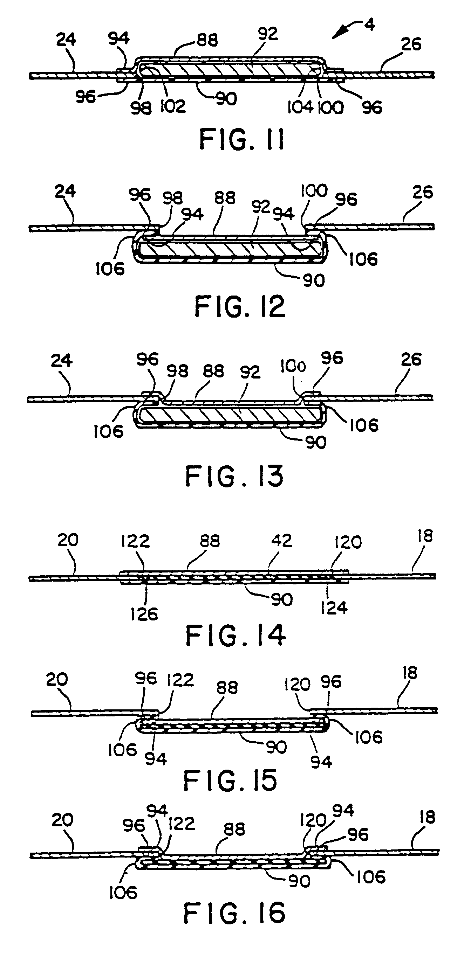

Referring now to Figure 11, which is a cross-section through Figure 5, the attachment of waste containment section 4 with stretchable side members 18, 20, 24, 26 will be described. Waste containment section 4 generally comprises a liquid pervious bodyside liner 88, a liquid impervious outer cover 90 and an absorbent medium 92 between liner 88 and cover 90. Outer cover 90 can be a woven or nonwoven material, films, or a film-coated nonwoven material comprising cast or blown films of polyethylene, polypropylene, polyester or blends thereof. Outer cover 90 may also be a composite of a bonded carded or spunbonded or meltblown material, for example, a spunbonded-meltblown composite of thermoplastic material or a spunbonded-meltblown-spunbonded thermoplastic material, wherein the spunbonded layer can provide a cloth-like texture and the meltblown layer can provide liquid impermeability. Materials of which outer cover 90 can be made include nonwovens having a high basis weight, such as about 10 grams per square meter (0.4 ounces per square yard) or basis weights greater than the aforementioned.

Outer cover 90 can also be extruded films of polyolefin polymers or copolymers, or other thermoplastic materials. Generally outer cover 90 will have a length from about 30.5 cm to about 76.2 cm (about 12 inches to about 30 inches), and a width from about 7.6 cm to about 50.8 cm (about 3 inches to about 20 inches).

Bodyside liner 88 can be a woven material, or a nonwoven material such as any flexible porous sheet of polyolefin fibers, such as polypropylene or polyethylene or polyester fibers; a web of spunbonded polypropylene or polyethylene or polyester fibers; a web of rayon fibers; a bonded carded web of synthetic or natural fibers or blends thereof. Liner 88 can also be an apertured plastic film. Liner 88 generally will have a length from about 30.4 cm to 76.2 cm (about 12 inches to about 30 inches), and a width from about 7.6 cm to about 50.8 cm (about 3 inches to about 20 inches).

Absorbent medium 92 can be made of wood pulp fluff or a mixture of wood pulp fluff and a superabsorbent material, or a wood pulp fluff integrated with a thermoplastic absorbent material treated with a surfactant. Thermal binders, such a Pulpex® can be used in blends or layering with the fluff and superabsorbent. Medium 92 can also be a batt of meltblown synthetic fibers, a bonded carded web of synthetic or natural fibers or blends thereof, a composite of meltblown fibers and the like. The synthetic fibers can be, but are not limited to, polypropylene, polyethylene, polyester and copolymers of these or other polyolefins. Medium 92 generally will have a length from about 7.6 cm to about 76.2 cm (about 3 inches to about 30 inches), and a width from about 7.6 cm to about 50.8 cm (about 3 inches to about 20 inches).

As illustrated in Figure 11, outer cover 90 and bodyside liner 88 sandwich absorbent medium 92, which is preferably adhered only to outer cover 90 by any suitable adhesive or other means. Alternatively, absorbent medium 92 could be joined to bodyside liner 88 or both bodyside liner 88 and outer cover 90. The longitudinal edge portions 94 of bodyside liner 88 and the longitudinal edge portions 96 of outer cover 90 also sandwich respective edge portions of stretchable side members 24, 26 to join them to waste containment section 4. Stretchable side members 24, 26 can be joined or adhered between respective edge portions 94, 96 by heat sealing, ultrasonic sealing, adhesive sealing or by other conventional means, such as stitching and the like.

As illustrated in Figure 11, stretchable side members 24, 26 have respective inner sides 98, 100 that are illustrated as being just slightly spaced apart from respective absorbent sides 102, 104. One of the unique features of the present invention is the positional relationship between inner sides 98, 100 and absorbent sides 102, 104. Depending on the degree of elasticity and the amount of gathering desired, inner sides 98, 100 can be positioned at different distances from respective absorbent sides 102, 104. For example, inner sides 98, 100 can be in direct abutment against absorbent sides 102, 104 to provide maximum gathering, or inner sides 98, 100 can be spaced apart from respective absorbent sides 102, 104 as desired. A desired range of distances between inner sides 98, 100 and respective absorbent sides 102, 104 is about 0 cm to about 5.1 cm (about 0 inches to about 2 inches). A preferred range of distance between inner sides 98, 100 and absorbent sides 102, 104 is about 0 cm to about 2.54 cm (about 0 to about 1 inch) and a more preferred distance is about 0 cm to about 1.3 cm (about 0 to about 1/2 inch).

When inner sides 98, 100 are in abutment against absorbent sides 102, 104, the effect is to provide additional seal against leakage, and to allow for a more uniform transition from side panel to absorbent.

As inner sides 98, 100 are spaced an increasing distance from absorbent sides 102, 104, the resulting effect is to allow additional flexibility to the leg gasketing at each leg opening.

Referring to Figure 12, there is illustrated a modification to the structure of Figure 11. The longitudinal edge portions 94 of bodyside liner 88 terminate substantially at absorbent sides 102, 104. Longitudinal edge portions 96 of outer cover 90 overlap absorbent sides 102, 104 and edge portions 94 to form liquid-impervious baffles 106. Stretchable side members 24, 26 are then joined on top of edge portions 96, which form baffles 106, such that edge portions 96 of outer cover 90 are joined between respective stretchable side members 24, 26 and bodyside liner 88. As illustrated in Figure 12, inner sides 98, 100 are substantially coincident with the remote ends of edge portions 94. If desired for better fluid control, baffles 106, which again are the overlapping edge portions 96 of outer cover 90, can extend further inwardly over bodyside liner 88 and beyond inner sides 98, 100, thereby creating larger baffles 106. By so extending baffles 106 toward the central portion of absorbent medium 92, there is a reduction in the amount of fluid flowback that may occur in both the longitudinal and transverse directions, thereby further reducing the chance of any fluid leakage about the leg openings 12, 14.

Referring to Figure 13, another modification of Figure 11 is illustrated. In this particular modification, edge portions 96 of outer cover 90 overlap only absorbent medium 92. Stretchable side members 24, 26 are then attached to the top of edge portions 96, again which form baffles 106, and bodyside liner 88 is then attached to stretchable side panels 24, 26. As illustrated in Figure 13, inner sides 98, 100 are substantially coincident with the ends of edge portions 96. However, edge portions 96, forming baffles 106, can extend further inwardly toward the center of absorbent medium 92, thereby providing greater protection against fluid flowback in both the longitudinal and transverse directions.

The percentage of overlap or coverage of absorbent medium 92 with baffles 106 can be 0 to about 99%, preferably about 10% to about 50%, and more preferably about 10% to about 20%.

As baffles 106 are disposed further inwardly toward the center of absorbent medium 92, inner sides 98, 100 can likewise be extended further inwardly before being joined to edge portions 96. The percent overlap or coverage of panel inner sides 98, 100 with absorbent medium 92 can be 0 to about 50%, preferably about 3% to about 20%, and more preferably about 6% to about 12%.

With reference to Figures 12, 13, side panels 24, 26 alternatively can be attached to the bottom surface of outer cover 90.

As described above, waste containment section 4 of absorbent garment 2 is maintained in a snug-fitting, comfortable fashion against the wearer by elastic or stretchable side panels 6, 8. The effect of side panels 6, 8, and leg elastics 46 extending about the inner portion of the wearer's legs, is to provide not only vertical forces that maintain the waste containment section 4 against the wearer's crotch area, but also inwardly directed horizontal force vectors against the hips and mid-section that hold or hug waste containment section 4 against the sides of the wearer, both before and after a void.

Referring to Figure 5, waist elastics 42, 44 are illustrated with absorbent garment 2 in a two-dimensional or planar form before the construction of seams 30, 32. Ends 108, 110 of absorbent medium 92 terminate short of outer cover ends 112, 114 and bodyside liner ends 116, 118. Absorbent ends 108, 110 are spaced a distance from outer cover ends 112, 114 in the range of about 1/2 inch to about 2 inches. Generally, bodyside liner ends 116, 118 are substantially coincident with outer cover ends 112, 114, and the total garment length is measured between these ends. It may be that bodyside liner ends 116, 118 extend beyond outer cover ends 112, 114 and are folded over ends 112, 114 to form a skirt or fringe about waist opening 10. In this case, the total garment length is measured between ends 112, 114. Similarly, outer cover ends 112, 114 could extend beyond bodyside liner ends 116, 118 and be folded thereover, and the total garment length is measured between ends 116, 118.

Preferably, waist elastics 42, 44 are made of an activatable elastic material applied in an unstretched condition. Thereafter, waist elastics 42, 44 are activated, such as by heat, light, moisture or the like, so as to retract and become elastic. Types of these activatable elastic materials can be purchased from the Minnesota Mining and Manufacturing Company.

Each waist elastic 42, 44 can be a single ribbon of elastic material that is suitably adhered solely to bodyside liner 88, or to outer cover 90, or to both liner 88 and cover 90. A single ribbon of waist elastic 42 or 44 in the relaxed, attached condition has a length of about 5.1 cm to about 30.5 cm (about 2 inches to about 12 inches) and a relaxed, attached width of about 0.3 cm to about 5.1 cm (about 1/8 inch to about 2 inches). Generally, waist elastics 42, 44 will be adhered in a stretched condition, and in the stretched condition, each waist elastic 42, 44 will have a stretched length of about 5.7 cm to about 38.1 cm (about 2 1/4 inches to about 15 inches) and a stretched width of about 0.3 cm to about 4.8 cm (about 1/8 inch to about 1 7/8 inches). These parameters should provide a relaxed, attached length of about 50% to about 100% of the width of waste containment section 4.

Instead of each waist elastic 42, 44 being a single ribbon of elastic material, each may be comprised of a multiple strand of ribbons having a generally rectangular cross-section or ropes having a generally circular cross-section. For example, if each waist elastic 42, 44 comprises multiple strands of ribbons, each of the ribbons in the strand will have a length similar to that for a single ribbon and a width from about 0.3 cm to about 1.9 cm (about 1/8 inch to about 3/4 inch). If each waist elastic 42, 44 comprises multiple strands of rope elastics, each rope preferably has a length similar as above and a width or diameter from about 0.1 cm to about 0.6 cm (about 0.04 inch to about 0.25 inch).

Waist elastics 42, 44 may be made of any suitable elastic material, such as those of which side panels 6, 8 or stretchable layer 86 can be made. Suitable adhesives for adhering waist elastics 42, 44 to absorbent garment 2 include hot melt adhesives, spray adhesives, self-adhering elastomeric materials and the like.

Referring now to Figures 14-16, the various seam configurations for waist elastics 42, 44 will be described. Since both waist elastics 42, 44 can be attached in a similar manner, only a description with reference to waist elastic 42 will be made with the understanding that it also applies to waist elastic 44. In Figure 14, waist elastic 42 is sandwiched between bodyside liner 88 and outer cover 90. Similarly, stretchable side members 18, 20 are sandwiched between bodyside liner 88 and outer cover 90, with inner sides 120, 122 abutting against respective waist elastic sides 124, 126. The attachment of waist elastic 42 and stretchable side members 18, 20 to bodyside liner 88 and outer cover 90 can be made by heat sealing, ultrasonic sealing, adhesive sealing or any other suitable means. In Figure 14, inner sides 120, 122 abut against respective waist elastic sides 124, 126 in order to provide a continuous stretchable or elastic effect about the periphery of waist opening 10. However, inner sides 120, 122 can be spaced from respective waist elastic sides 124, 126 in the range of about 0 cm to about 5.1 cm (about 0 inches to about 2 inches). A preferred range is from about 0 cm to about 2.54 cm (about 0 inches to about 1 inch), and a more preferred range is from about 0 cm to about 1.3 cm (about 0 inches to about 1/2 inch). Also, side panel inner sides 120, 122 could overlap partially or completely waist elastics 42, 44 in that area between ends 116, 108 and ends 118, 110.

Referring now to Figure 15, the sides of waist elastic 42 and bodyside liner 88 are substantially coincident, and the longitudinal edge portions 96 of outer cover 90 are folded to overlap longitudinal edge portions 94 of bodyside liner 88, thereby forming baffles 106. Stretchable side members 18, 20 are then adhered to the exposed tops of baffles 106, such that inner sides 120, 122 are substantially coincident with the ends of baffles 106. Baffles 106 can be extended further inwardly toward the center portion of bodyside liner 88 and absorbent medium 92. The percent of overlap or coverage of baffles 106 with absorbent medium 92 can be in the range of about 0% to about 90%. Preferably, the coverage is about 5% to about 50% and more preferably from about 8% to about 13%.

Referring now to Figure 16, longitudinal edge portions 96 of outer cover 90 overlap waist elastic 42 to form baffles 106, and stretchable side members 18, 20 are then adhered to baffles 106. Bodyside liner 88 is disposed over waist elastic 42 and inner sides 120, 122 of stretchable side members 18, 20. Baffles 106 can extend over elastic 42 as described above with reference to Figure 15.

Referring now to Figure 5, leg elastics 46 may be made of the same or other described materials of which waist elastics 42, 44 can be made. Leg elastics 46 may be similarly adhered by one of those methods described for adhering waist elastics 42, 44. Each leg elastic 46 is preferably a single ribbon of elastic material having a relaxed, attached length of about 2.54 cm to about 45.7 cm (about 1 inch to about 18 inches) and a relaxed, attached width of about 0.3 cm to about 7.6 cm (about 1/8 inch to about 3 inches), and an elongation of about 25% to about 350%. A preferred length is about 5.1 cm to about 22.9 cm (about 2 inches to about 9 inches) and an elongation of about 30% to about 260%. A more preferred length is about 7.6 cm to about 10.2 cm (about 3 inches to about 4 inches) and an elongation of about 125% to about 200%. A preferred relaxed width is about 0.6 cm to about 3.8 cm (about 1/4 inch to about 1 1/2 inches) and a more preferred width is about 1.3 cm to about 2.5 cm (about 1/2 inch to about 1 inch).

As a percentage of total garment length, the relaxed, attached elastic 46 has a length of about 10% to about 100% of total garment length. A preferred length is about 10% to about 50%, and a more preferred length is about 15% to about 25%.

As with waist elastics 42, 44, leg elastics 46 do not necessarily need to be a single ribbon of elastic material, but can be multiple strands of ropes or ribbons of elastic material.

If elastics 46 are rope-like, preferred diameters are between about 0.1 cm to 0.6 cm (about 0.04 inches to about 0.25 inches).

Referring to Figure 5A, leg elastics 46 are positioned between longitudinal edge portions 94 of bodyside liner 88 and longitudinal edge portions 96 of outer cover 90. Leg elastics 46 can abut against or be spaced apart from respective absorbent sides 102, 104.

Figure 5B illustrates a modification of the placement of leg elastics 46. In this modification, outer cover 90 is wrapped around absorbent edges 102, 104 so as to overlap and form baffles 106. Leg elastics 46 are then positioned on top of baffles 106, which are also longitudinal edge portions 96, and bodyside liner 88 is then disposed over leg elastics 46.

Similarly, Figure 5C illustrates leg elastics 46 being positioned on top of longitudinal edge portions 94 of bodyside liner 88, and with outer cover 90 then overlapping leg elastics 46 to form baffles 106.

Also, in both modifications illustrated in Figures 5B and 5C, leg elastics 46' may be positioned below absorbent medium 92, as illustrated in dashed lines in Figure 5C. In this case, leg elastics 46' would be positioned between outer cover 90 and absorbent medium 92. In Figure 5A, leg elastics 46 can also be positioned either above or below absorbent medium 92.

Referring now to Figure 6, there is illustrated a modification of the embodiment in Figure 1 wherein waist elastics 42, 44 and nonstretchable intermediate members 22, 28 are absent. Also, absorbent medium 92 extends to and is substantially coincident with outer cover ends 112, 114 and bodyside liner ends 116, 118. In all other respects, the modification in Figure 6 is identical to Figure 5. The absorbent garment 2 formed from the modification in Figure 6 would be the same as that illustrated in Figure 1, but without the waist elastics 42, 44 and the intermediate members 22, 28. This modification can be used for smaller torsos because of the removal of intermediate members 22, 28 or, if additional elasticity is desired, then intermediate members 22, 28 are replaced by extending stretchable side members 18, 20, 24, 26.

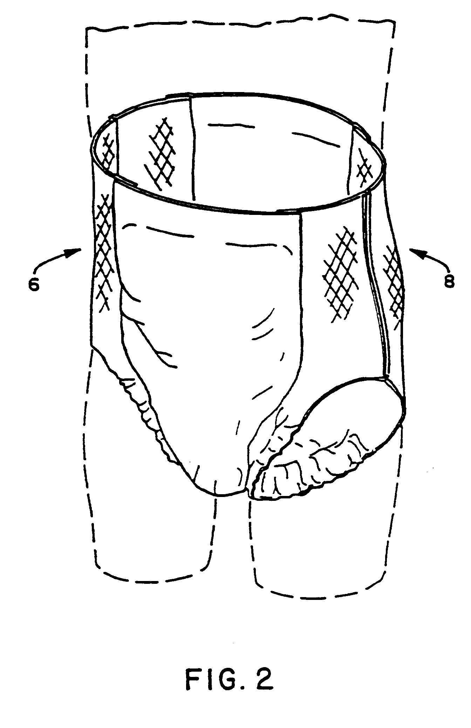

Another embodiment is illustrated in Figures 2, 4, 7 and 8 wherein leg elastics 46 have been replaced by side panels 6, 8, and intermediate members 22, 28 and waist elastics 42, 44 are absent. Each side panel 6, 8 is illustrated in Figure 7 as comprising three areas indicated as X, Y, and Z. Those areas X, Y of side panels 6, 8 are attached to waste containment section 4 in a relaxed condition, while area Z of side panels 6, 8 are prestretched before being joined to waste containment section 4. The area Z is disposed intermediate the longitudinal ends of waste containment section 4, and upon being relaxed, the garment tends to assume the body-conforming shape illustrated in Figure 8. Area Z can also be attached in a relaxed condition. Also area Z can have a width narrower than areas X and Y.

The present invention also contemplates use of leg elastics 46 in conjunction with side panels 6, 8 in the embodiment of Figures 2, 4, 7, and 8. Leg elastics 46 can abut, overlap, or be spaced apart from respective panels 6, 8.

As explained above, the garment of the present invention is designed to fit a large range of sizes merely by changing the dimensions of elastic side panels 6, 8, or by changing the type of elastic material of which side panels 6, 8 are made. Generally, the ranges of sizes can be varied by (1) selecting a material having a desired modulus of elasticity and/or (2) by increasing the length and width dimensions of a given elastic material of which side panels 6, 8 are made. Side panels 6, 8 will generally have a width of about 0.1 cm to about 12.7 cm (about 1/2 inch to about 5 inches), and will be made of a material having an elongation or elasticity from about 10% to about 500%. Preferably, side panels 6, 8 will have a width from about 5.1 cm to about 8.9 cm (about 2 inches to about 3 1/2 inches), and the material of which they are made will have an elasticity between about 50% to about 300%. In a more preferred embodiment, side panels 6, 8 will have a width of about 3.2 cm to about 5.1 cm (about 1 1/4 inches to about 2 inches) and an elasticity from about 75% to about 200%.

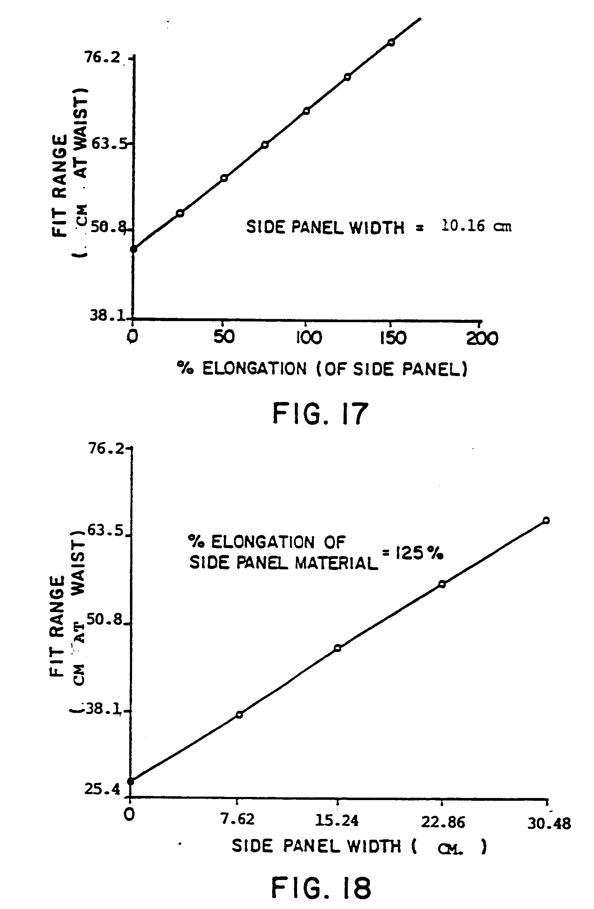

Referring to Figures 17-19, the relationships between side panels 6, 8 and the range of sizes of absorbent garment 2 are graphically illustrated. In Figure 17, each side panel 6, 8 has a width of about 10.2 cm (about 4 inches), and the percent elongation of the side panels is plotted against the fit range in inches at the waist.

As illustrated, there is a generally linear relationship between the percent elongation of the elastic material of which side panels 6, 8 are made and the size range of the waist measured in cm (inches).

Figure 18 illustrates the relationship between side panel width and a fit range in cm (inches) at the waist for an elastic material having a percent elongation of 125%. As illustrated, there is a generally linear relationship between an increase in the side panel width cm (inches) versus the fit range in cm (inches) at the waist.

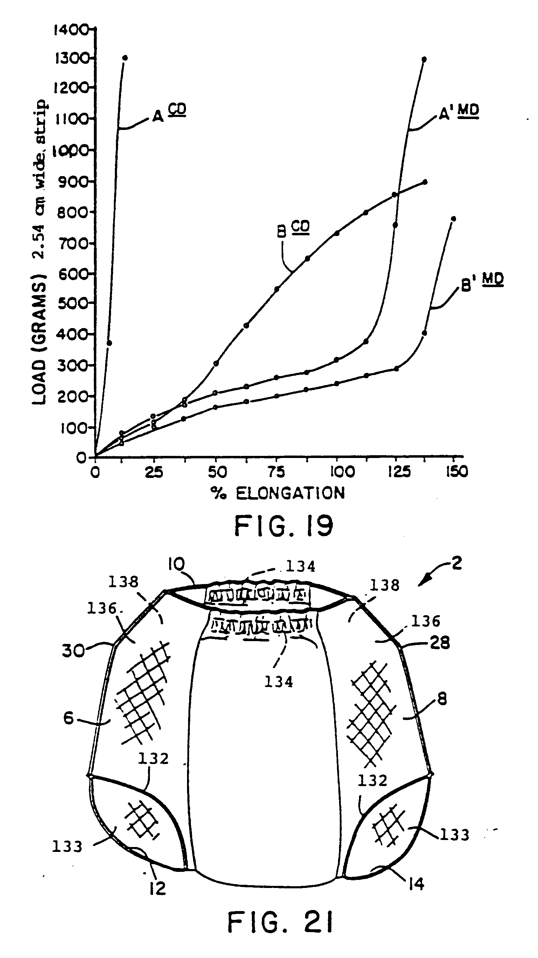

Figure 19 is a graph plotting percent elongation versus the load in grams on a 2.5 cm (1 inch) wide strip of elastic material. The load in grams measures the tension at elongation of the particular material, and this feature is maximized by theoretically having a slope of 0 for each plot. In Figure 19, the plotted curves represent two elastic materials, wherein curve A represents the cross-direction stretch and curve A' represents the machine-direction stretch of one material; and curve B represents the cross-direction stretch and curve B' represents the machine-direction stretch of the other material. The machine-direction stretch, preferably in the stretch-bonded laminate embodiment, is the force vector applied horizontally inwardly or toward the hips and mid-section when worn. This stretch-strain relationship is important to the use and performance of the garment of the present invention. The side panel material must stretch to adjust to various sizes. The tension cannot be so high that the garment is difficult to use or be too tight during use. Nor, can it be so low in tension as not to maintain the product in position during use. Preferably, the materials of which side panels 6, 8 are made will have a tension range, i.e., load (grams) per 2.5 cm (1 inch) wide strips, from about 50 grams to about 1,000 grams. In a more preferred embodiment, the side panel materials would have a tension range of about 200 to about 500 grams per 1 inch wide strip. Secondly, curves A' and B' both illustrate relatively very gradual slopes between 0 and about 125% elongation or elasticity. The slope is important to maintain a constant fit tension at the various sizes. With a theoretical slope of 0, the tension of the product would be the same at the relaxed size as it would be at the fully-stretched size.

Referring now to Figure 21, there is yet another modification of absorbent garment 2 that includes waist elastics 134. A unique feature of this modification of absorbent garment 2 is the geometry of elastic side panels 6, 8. Specifically, it can be seen that the front portions 132 of leg openings 12, 14 are cut higher than the back portions 133 of leg openings 12, 14. The purpose for this particular geometry of leg openings 12, 14 is to improve further the fit of the garment. The added material in the back provides coverage of the buttocks, while in the front the cut is higher in following the curvature of the leg, thereby permitting freer leg movement.

The upper portions of elastic side panels 6, 8 are identified as remote end segments 136, 138, and they are cut so that they slope inwardly and upwardly from the intermediate portions of side panels 6, 8 toward waist opening 10. The purpose for this is to improve further the fit of the garment, especially when the user is disproportionate at the hips and waist. The design or configuration also assists in pulling the garment up in place. The length of each sloping end segment 136, 138 is from about 3% to about 40% of total garment length, preferably about 5% to about 25% of total garment length, and more preferably about 10% to about 15% of total garment length.

The angular slope, as measured with the vertical in Figure 21, of end segments 136, 138 is from about 5° to about 55°, preferably from about 10° to about 40°, and more preferably from about 15° to about 30°.

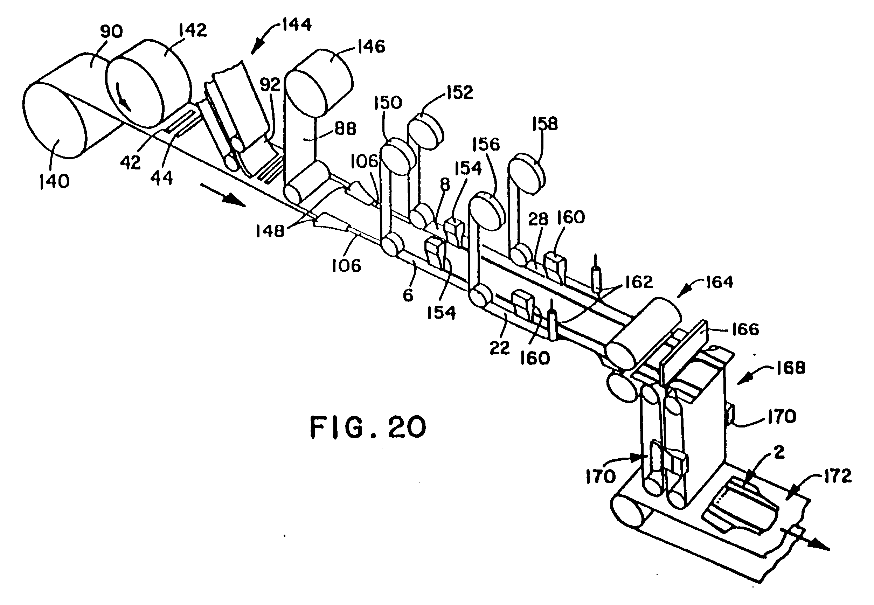

Referring now to Figure 20, a description will be made of one process for making one embodiment of garment 2. Supply roll 140 provides a continuous supply of outer cover 90 to supply drum 142, which attaches, if desired, waist elastics 42, 40 thereon. After application of waist elastics 42, 44, outer cover 90 continues to conveyor assembly 144 which positions absorbent medium 92 between waist elastics 42, 44. Thereafter, supply roll 146 delivers a continuous supply of bodyside liner 88 on top of waist elastics 42, 44, absorbent medium 92 and the continuous supply of outer cover 90. Folding bars 148 then fold outer cover 90, which has a width greater than the width of absorbent medium 92 and bodyside liner 88 to form baffles 106. After baffles 106 have been formed, supply rolls 150, 152 provide a continuous supply of elastic side panels 6, 8, and bonding station 154 then bonds, such as by ultrasonic, thermal, or adhesive bonding, elastic side panels 6, 8, bodyside liner 88 and outer cover 90. Similarly, supply rolls 156, 158 provide a continuous supply of intermediate members 22, 28, and bonding station 160 then bonds, such as by ultrasonic, thermal, or adhesive bonding, elastic side panels 6, 8 and intermediate members 22, 28. Leg cutout station 162, which can be pressurized fluid-jets or a rotary die cutter, then cuts side panels 6, 8 to form leg openings 12, 14. As the composite continues through the process, cutting station 164 severs the composite, which is then tucked or folded in half by tucker bar 166, which contacts an intermediate portion of a severed composite and moves it between the individual conveyors of conveyor assembly 168. Located in conjunction with conveyor assembly 168 is seam bonding station 170 which bonds, such as by ultrasonic, thermal, or adhesive bonding, elastic side panels 6, 8 to form seams 30, 32 of absorbent garment 2. Conveyor assembly 168 then delivers absorbent garment 2 to transfer conveyor assembly 172, which delivers absorbent garments 2 to the next handling station.

The process illustrated in Figure 20 can be easily adapted to make other embodiments of the present invention such as that illustrated in Figure 2, and other constructions such as those illustrated in Figures 11-13.

In the following specific aspects of the invention are described. According to one aspect the invention provides a disposable pant-like garment 2 for absorbing human discharge comprising an absorbent assembly comprising a liquid-impervious outer cover 90, a liquid pervious liner 88 and an absorbent medium 92 therebetween said absorbent assembly further comprising generally opposite side edges and generally opposite end edges, a pair of stretchable side panels 6, 8, being joined to each one of said side edges to form with said absorbent assembly a waist opening 10, and a pair of leg openings 12, 14, and a gathering means 46 being joined along at least a portion of a respective one of each said leg openings 12, 14, for gathering said portions; whereby said stretchable side panels 6, 8, provide generally inwardly directed force vectors against a wearer to maintain said garment 2 snugly against the wearer's body 16 and said absorbent assembly snugly in place against the crotch area both before and after a discharge, and said gathering means 46 provide elasticity about said leg openings 12, 14, to prevent leakage thereat.

Gathering means may have a relaxed, attached length of 10% to 100%, preferably 10% to 50% and more preferably 15% to 5% of the total length of said garment. Said gathering means may also have an elasticity of 25% to 350% preferably 30% to 260% and more preferably 125% to 200%. Further, each said gathering means 46 may be a plurality of elongate elastic members and/or each said gathering means 46 may be prestretched when joined to its respective said leg opening.

In the aforementioned garment, each said gathering means 46 may be an integral extension of at least one of said stretchable side panels 6, 8, wherein preferably each said integral extension is prestretched when joined to said absorbent assembly. The aforementioned garment may further comprise a pair of substantially nonstretchable side sections 22, 28, each said side section being joined intermediate of a respective said stretchable side panel 6, 8. At least one of said stretchable side panels 6, 8, may further be manually tearable from said absorbent assembly. Each said stretchable side panel may also be stretch-bonded laminate 80.

Said stretch-bonded laminate 80 may comprise at least two layers 82, 84, 86, one of said layers being made of an elastomeric material or said stretch-bonded laminate 80 may comprise three layers 82, 84, 86, the middle layer 86 being made of an elastomeric material.

In the aforementioned garment, at least an edge portion of each gathering means 46 may be joined between said outer cover 90 and said liner 92, wherein said gathering means 46 edge portions preferably directly abut against respective sides of said absorbent medium 92, or wherein said gathering means 46 edge portion may be spaced apart from respective sides of said absorbent medium 0 cm to 5.08 cm (0 inches to 2 inches), preferably 0 cm to 2.54 cm (0 inches to 1 inch), and more preferably 0 cm to 1.27 cm (0 inches to 1-2 inch), or wherein each said gathering means 46 edge portion may extend over a respective side of said absorbent medium a distance of 0% to 50%, preferably 3% to 20% and more preferably 5% to 12% of the maximum width of said absorbent medium.

In the aforementioned garment, each said side panel 6, 8, may have a tension range per 2.54 cm (inch) of 50 grams to 1000 grams, preferably 200 grams to 500 grams. Further in said garment each side panel may have an elasticity of 10% to 500%, preferably 50% to 300% and more preferably 75% to 200%.

Further in the aforementioned garment said side panels 6, 8, may comprise between 20% to 80%, preferably 25% to 50% and more preferably 35% to 45% of the total surface area of said garment 2.

In the aforementioned garment, the width of each said gathering means 46 may be between 1% to 100%, preferably 20% to 80% and more preferably 30% to 50% of the maximum width of its respective said side panel 6, 8.

Further in said garment said outer cover 90 may comprise at least two layers, the outermost one of said layers being made of a cloth-like material and the innermost one of said layers being made of a liquid-impervious material.

The aforementioned garment may further comprise a waist elastic 44 at least at one end portion of said absorbent assembly, wherein said waist elastic 44 preferably comprises a plurality of elastic members.

According to a further aspect, the invention provides a disposable pant-like garment for absorbing human discharge, comprising an absorbent assembly comprising a liquid-impervious outer cover 90, a liquid-pervious liner 88 and an absorbent medium 92 therebetween. The said absorbent assembly further comprises generally opposite side edges and generally opposite end edges, a pair of stretchable side panels 6, 8 being joined to each one of said side edges to form with said absorbent assembly a waist opening and a pair of leg openings 12, 14, each said pair of stretchable side panels being generally peripherally disposed about a portion of a respective one of said leg openings and a gathering means 46 being joined generally along the remaining portion of each said leg opening 12, 14, whereby each said pair of stretchable side panels and its respective said gathering means 46, are generally peripherally disposed about a respective said leg opening 12, 14, to provide elasticity thereto and to prevent leakage thereat, and said stretchable side panels 6, 8, provide generally inwardly directed force vectors against a wearer to maintain said garment snugly against the wearer's body 16 both before and after a discharge.

In the aforementioned garment an end segment portion 136, 138 of at least one of said stretchable side panels 6, 8, may be angularity disposed generally inwardly toward said waist opening 10, wherein the length of said end segment portion 136, 138, is 3% to 40%, preferably 5% to 25% and more preferably 10% to 15% of the total length of said garment.

In this garment said end segment portion may angle inwardly toward said waist opening (10) between 5° to 55°, preferably 10° to 40° and more preferably 15° to 30°.

Further the front edge portion 132 of each said leg opening 12, 14, may be nearer to said waist opening 10 than the back edge portion 133.

According to a still further aspect the invention provides disposable pant-like garment for absorbing human discharge, comprising an absorbent assembly comprising a liquid impervious outer cover 90, a liquid pervious liner 88, and an absorbent medium 92 therebetween, a pair of stretchable side panels 6, 8, being joined to said absorbent assembly to form a waist opening 10 and a pair of leg openings 12, 14, and an intermediate portion of each said stretchable side panel 6, 8, being generally peripherally disposed along a crotch portion of a respective one of said leg openings, whereby said stretchable side panels provide generally inwardly directed force vectors against a wearer, to maintain said garment snugly against the wearer's body 16 and said absorbent assembly snugly in place against the crotch area both before and after a discharge.

In the aforementioned garment the width of each said intermediate portion may be between 5% to 100% of the maximum width of its respective said side panels, or each said intermediate portion is prestretched before being joined.

Further in the aforementioned garment an edge portion of each said side panel may be joined between said outer cover 90 and said liner 88, wherein the said edge portions preferably directly abut against respective sides of said absorbent medium 92 or wherein said edge portions are spaced apart from respective sides of said absorbent medium 92 from about 0 cm to about 5.08 cm (about 0 in. to about 2 in.).

In the aforementioned garment each said side panel 6, 8 may extend over a respective side of said absorbent medium 92 a distance of 0% to 50% of the maximum width of said absorbent medium 92. Also said outer cover 90 may comprise at least two layers, the outermost one of said layers being made of a cloth-like material and the innermost one of said layers being made of a liquid-impervious material, wherein the said outermost layer preferably is a spunbonded thermoplastic material or a bonded carded web of thermoplastic material or a liquid impervious meltblown thermoplastic material or an extruded film of thermoplastic material.

In the aforementioned garment each said side panel 6, 8 may be a stretch-bonded laminate. Also at least one seam may be manually tearable.

In the aforementioned garment each said side panel 6, 8 may also have a tension range per 2.54 cm (inch) of 50 grams to 1000 grams, each said side panel 6, 8, may have an elasticity of 10% to 500%.

Further said garment may comprise a waist elastic 42 at each end portion of said absorbent assembly wherein said waist elastic 42 preferably is an activatable elastic material. According to a still further aspect the invention provides a disposable pant-like garment 2 for absorbing human discharge, comprising an absorbent assembly comprising a liquid impervious outer cover 90, a liquid pervious liner 88 and an absorbent medium 92 therebetween , a pair of stretchable side panels 6, 8, being joined to said absorbent assembly to form a waist opening and a pair of leg openings 12, 14, an intermediate portion of each said stretchable side panel 6, 8, being generally peripherally disposed along a crotch portion of a respective one of said leg opening and an end segment portion 136, 138, of each said stretchable side panel 6, 8, being angularly disposed generally inwardly toward said waist opening 10, thereby to provide a better fit at the waist of the wearer 16, whereby said stretchable side panels 6, 8, provide generally inwardly directed force vectors against the wearer 16 to maintain said garment 2 snugly against the wearer 16 and said absorbent assembly snugly in place against the crotch area both before and after a discharge.

In the aforementioned garment the length of each said end segment portion 136, 138, may be 3% to 40% of the total length of said garment.

Further each said end segment portion 136, 138 may angle inwardly toward said waist opening 10, at an angle between 5° to 55°.

The front edge portion 132 of each said leg opening 12, 14 may be nearer to said waist opening 10 than the back edge portion 133.

Also said side panels 6, 8, may comprise between 20% to 80% of the total surface area of said garment 2. Further the width of each said intermediate portion may be between 5% to 100% of the maximum width of its respective said side panel 6, 8.

In the aforementioned garment an edge portion of each said side panel 6, 8, may be joined between said outer cover 90 and liner 88, wherein said edge portion of each said side panel 6, 8, preferably abuts directly against said absorbent medium 92 or is spaced apart from said absorbent medium from 0 cm to 5.08 cm (0 in. to 2 in.).

In the aforementioned garment each said side panel 6, 8 may extend over said absorbent medium 92 a distance of 0% to 50% of the maximum width of said absorbent medium 92.

## Claims

1. A disposable pant-like garment (2) for absorbing human discharge, comprising:
an absorbent assembly comprising a liquid-impervious outer cover (90), a liquid-pervious liner (88) and an absorbent medium (92); said absorbent assembly further comprising generally opposite side edges and generally opposite end edges; and
gathering means (46) providing elasticity along at least portions (76,78) of a pair of leg openings (12,14) of said garment (2) to prevent leakage thereat,
characterized by
elastic stretchable side panels (6,8) joined to said side edges to form with said absorbent assembly a child's training pant having a waist opening (10) and said leg openings (12,14), whereby said elastic stretchable side panels (6,8) maintain said training pant (2) snugly against the wearer's body (16).

2. The garment of claim 1 wherein said gathering means has a relaxed, attached length of 10% to 100%, preferably 10% to 50%, and more preferably 150% to 25% of the total length of said garment.

3. The garment of claim 1 or 2 wherein said gathering means (46) has an elasticity of 25% to 350%, preferably 300% to 260% and more preferably 125% to 200%.

4. The garment of any one of claims 1 to 3 wherein each said gathering means (46) is a plurality of elongated elastic members.

5. The garment of any one of claims 1 to 4 wherein each said gathering means (46) is pre-stretched when joined to its respective said leg opening.

6. The garment of any one of claims 1 to 4 wherein each said gathering means (46) is an integral extension of one of said elastic stretchable side panels (6,8).

7. The garment of claim 6 wherein each said integral extension is pre-stretched when joined to said absorbent assembly.

8. The garment of any one of claims 1 to 7 further comprising a pair of substantially non-stretchable side sections (22,28), each said side section being joined intermediate of a respective said elastic stretchable side panel (6,8).

9. The garment of any one of the preceding claims wherein each of said elastic stretchable side panels (6,8) comprises two elastic stretchable side members (18,20,22,24).

10. The garment of claims 8 and 9 wherein said side sections (22, 28) are joined by seams (34,36,38,40) to the elastic stretchable side members (18,20, 24,26).

11. The garment of any one of the preceding claims wherein at least one of said elastic stretchable side panels (6,8) is manually tearable from said absorbent assembly.

12. The garment of any one of the preceding claims wherein said gathering means (46) edge portions directly abut against respective sides of said absorbent medium (92).

13. The garment of any one of the preceding claims wherein said gathering means (46) edge portions are spaced apart from respective sides of said absorbent medium 0 cm to 5.08 cm (0 inches to 2 inches), preferably 0 cm to 2.54 cm (0 inches to 1 inch), and more preferably 0 cm to 1.27 cm (0 inches to 1/2 inch).

14. The garment of any one of the preceding claims wherein each said gathering means (46) edge portion extends over a respective side of said absorbent medium a distance of 0% to 50%, preferably 3% to 20%, and more preferably 6% to 12% of the maximum width of said absorbent medium.

15. The garment of any one of the preceding claims wherein each said elastic stretchable side panel (6,8) has a tension range per 2.54 cm (per inch) of 50 grams to 1,000 grams, preferably 200 grams to 500 grams.

16. The garment of any one of the preceding claims wherein the width of each said gathering means (46) is between 1% to 100%, preferably 20% to 80%, and more preferably 30% to 50% of the maximum width of its respective said side panel (6,8).

17. The garment of any one of the preceding claims further comprising a waist elastic (44) at least at one end portion of said absorbent assembly.

18. The garment of claim 17 wherein said waist elastic (44) comprises a plurality of elastic members.

19. The garment of claim 1 wherein each of said elastic stretchable side panels (6,8) comprises an intermediate portion (Z), said intermediate portion (Z) being generally peripherally disposed along a crotch portion of a respective one of said leg openings (12,14), whereby said stretchable side panels (6,8) provide generally inwardly directed force vectors against a wearer for maintaining said garment (2) snugly against the wearer's body (16) and said absorbent assembly snugly in place against the crotch area both before and after a discharge.

20. The disposable garment of claim 19 wherein said elastic stretchable side panels (6,8) comprise stretchable side members (18,20,24,26).

21. The garment of claim 19 or 20, wherein the width of each said intermediate portion is between 5% to 100% of the maximum width of its respective said side panels (6,8).

22. The garment of claim 19 wherein each said intermediate portion is prestretched before being joined.

23. The garment of claim 19 wherein the inner sides (98,100) of elastic stretchable side members (24,26) directly abut against respective sides (102,104) of said absorbent medium (92).

24. The garment of claim 19 wherein the inner sides (98,100) of elastic stretchable side members (24,26) are spaced apart from respective sides (102,104) of said absorbent medium (92) from about 0 cm to about 5.08 cm (0 inches to about 2 inches).

25. The garment of any one of claims 19 to 24 wherein each said side panel (6,8) extends over a respective side of said absorbent medium (92) a distance of 0% to 50% of the maximum width of said absorbent medium (92).

26. The garment of any one of claims 19 to 25 wherein at least one seam (30,32) is manually tearable.

27. The garment of any one of claims 19 to 26 wherein each said side panel (6,8) has a tension range of 2.54 cm (1 inch) of 50 grams to 1,000 grams.

28. The garment of any one of claims 19 to 27 further comprising a waist elastic (42) at each end portion of said absorbent assembly.

29. The garment of claim 28 wherein said waist elastic (42) is an activatable elastic material.

30. The garment of any one of the preceding claims wherein each said side panel (6,8) is a stretch-bonded laminate (80).

31. The garment of claim 30 wherein said stretch-bonded laminate (80) comprises at least two layers (82,84,86), one of said layers (86) being made of an elastomeric material.

32. The garment of claim 30 wherein said stretch-bonded laminate (89) comprises three layers (82,84,86), the middle layer (86) being made of an elastomeric material.

33. The garment of any one of the preceding claims wherein said outer cover (90) comprises at least two layers, the outermost one of said layers being made of a cloth-like material and the innermost one of said layers being made of a liquid-impervious material.

34. The garment of claim 33 wherein said outermost layer is a spunbonded thermoplastic material.

35. The garment of claim 33, wherein said outermost layer is a bonded carded web of thermoplastic material.

36. The garment of claim 33 wherein said innermost layer is a liquid impervious meltblown thermoplastic material.

37. The garment of claim 33 wherein said innermost layer is an extruded film of thermoplastic material.

38. The garment of any one of the preceding claims wherein each said side panel (6,8) has an elasticity of 10% to 500%.

39. The garment of any one of claims 1 to 19, comprising an end segment portion (136,138) of each said side panel (6,8) being angularly disposed generally inwardly toward said waist opening (10), thereby to provide a better fit at the waist of the wearer (16).

40. The garment of claim 39 wherein the length of each said end segment portion (136,138) is 3% to 40% preferably 5% to 25% and more preferably 10% to 15% of the total length of said garment.

41. The garment of claim 39 or 40 wherein each said end segment portion (136,138) angles inwardly toward said waist opening (10) at an angle between 5° to 55°, preferably 10° to 40° and more preferably 15° to 30°.

42. The garment of one of claims 38 to 41 wherein the front edge portion (132) of each said leg opening (12,14) is nearer to said waist opening (10) than the back edge portion (133).

43. The garment of one of claims 38 to 42 wherein said side panels (6,8) comprise between 20% to 80% preferably 25% to 50% and more preferably 35% to 45% of the total surface area of said garment (2).

44. The garment of one of claims 38 to 43 wherein the width of each said intermediate portion is between 5% to 100% of the maximum width of its respective said side panel (6,8).

45. The garment of one of the preceding claims, wherein at least one seam (30,32) joins said elastic stretchable panels (6,8).

46. The garment of claim 1 wherein each of said side panels (6,8) includes two elastic stretchable side members (18,20,24,26) connecting an intermediate member (22,28) which is made of a nonstretchable material wherein both intermediate members (22,28) are made of two halves joined by respective seams (30,32) and an intermediate portion of each said stretchable side panel (6,8) being generally peripherally disposed along a crotch portion of a respective one of said leg openings (12,14), whereby said stretchable side panels (6,8) provide generally inwardly directed force vectors against a wearer for maintaining said garment snugly against the wearer's body (16) and said absorbent assembly snugly in place against the crotch area both before and after a discharge.

## Patentansprüche

1. Wegwerfbares höschenartiges Kleidungsstück (2) zum Absorbieren menschlicher Ausscheidungen, mit
einer absorbierenden Anordnung, die eine flüssigkeitsundurchlässige äußere Abdeckung (90), eine flüssigkeitsdurchlässige Einlage (88) und ein absorbierendes Medium (92) umfaßt; wobei die absorbierende Anordnung des weiteren im allgemeinen gegenüberliegende Seitenkanten und im allgemeinen gegenüberliegende Endkanten aufweist; und
einem Kräuselmittel (46) zur Bereitstellung von Elastizität zumindest entlang Abschnitten (76, 78) eines Paars von Schenkelöffnungen (12, 14) des Kleidungsstücks, um dort ein Auslaufen zu verhindern,
gekennzeichnet durch
elastische, dehnbare Seitenbahnen (6, 8), die mit den Seitenkanten verbunden sind, um mit der absorbierenden Anordnung ein Kinderhöschen für die Übergangszeit zum Sauberwerden mit einer Taillenöffnung (10) und den Schenkelöffnungen (12, 14) zu bilden, wobei die elastischen, dehnbaren Seitenbahnen (6, 8) das Höschen (2) für die Übergangszeit zum Sauberwerden enganliegend am Körper (16) des Trägers halten.

2. Kleidungsstück nach Anspruch 1, wobei das Kräuselmittel eine entspannte befestigte Länge von 10% bis 100%, vorzugsweise von 10% bis 50%, und noch bevorzugter von 15% bis 25%, der Gesamtlänge des Kleidungsstücks aufweist.

3. Kleidungsstück nach Anspruch 1 oder 2, wobei das Kräuselmittel (46) eine Elastizität von 25% bis 350%, vorzugsweise von 30% bis 260%, und noch bevorzugter von 125% bis 200%, aufweist.

4. Kleidungsstück nach einem der Ansprüche 1 bis 3, wobei jedes der Kräuselmittel (46) eine Mehrzahl von gedehnten elastischen Elementen aufweist.

5. Kleidungsstück nach einem der Ansprüche 1 bis 4, wobei jedes der Kräuselmittel (46) vorgedehnt ist, wenn es mit der jeweiligen Schenkelöffnung verbunden wird.

6. Kleidungsstück nach einem der Ansprüche 1 bis 4, wobei jedes der Kräuselmittel (46) ein integraler Fortsatz einer der elastischen dehnbaren Seitenbahnen (6, 8) ist.

7. Kleidungsstück nach Anspruch 6, wobei jeder der integralen Fortsätze vorgedehnt ist, wenn er mit der absorbierenden Anordnung verbunden wird.

8. Kleidungsstück nach einem der Ansprüche 1 bis 7, das ferner ein Paar im wesentlichen nicht-dehnbarer Seitenabschnitte (22, 28) aufweist, wobei jeder Seitenabschnitt in der Mitte einer entsprechenden elastischen, dehnbaren Seitenbahn (6, 8) angeordnet ist.

9. Kleidungsstück nach einem der vorhergehenden Ansprüche, wobei jede der elastischen dehnbaren Seitenbahnen (6, 8) zwei elastische dehnbare Seitenelemente (18, 20, 22, 24) aufweist.

10. Kleidungsstück nach einem der Ansprüche 8 und 9, wobei die Seitenabschnitte (22, 28) durch Säume (34, 36, 38, 40) mit den elastischen dehnbaren Seitenelementen (18, 20, 24, 26) verbunden sind.

11. Kleidungsstück nach einem der vorhergehenden Ansprüche, wobei mindestens eine der elastischen, dehnbaren Seitenbahnen (6, 8) manuell von der absorbierenden Anordnung abtrennbar ist.

12. Kleidungsstück nach einem der vorhergehenden Ansprüche, wobei die Kantenbereiche des Kräuselmittels (46) unmittelbar an den jeweiligen Seiten des absorbierenden Mediums (92) anliegen.

13. Kleidungsstück nach einem der vorhergehenden Ansprüche, wobei die Kantenbereiche des Kräuselmittels (46) 0 cm bis 5,08 cm (0 Inch bis 2 Inch), vorzugsweise 0 cm bis 2,54 cm (0 Inch bis 1 Inch), und noch bevorzugter 0 cm bis 1,27 cm (0 Inch bis 1/2 Inch) von den jeweiligen Seiten des absorbierenden Mediums beabstandet sind.

14. Kleidungsstück nach einem der vorhergehenden Ansprüche, wobei sich jeder Kantenbereich des Kräuselmittels (46) über eine jeweilige Seite des absorbierenden Mediums über eine Länge von 0% bis 50%, vorzugsweise 3% bis 20%, und noch bevorzugter 6% bis 12%, der maximalen Breite des absorbierenden Mediums erstreckt.

15. Kleidungsstück nach einem der vorhergehenden Ansprüche, wobei jede der elastischen, dehnbaren Seitenbahnen (6, 8) einen Spannungsbereich von 50 g bis 1000 g, vorzugsweise 200 g bis 500 g pro 2,54 cm (1 Inch) aufweist.

16. Kleidungsstück nach einem der vorhergehenden Ansprüche, wobei die Breite jedes Kräuselmittels (46) zwischen 1% und 100%, vorzugsweise 20% und 80%, besonders bevorzugt zwischen 30% und 50% der maximalen Breite der jeweiligen Seitenbahn (6, 8) beträgt.

17. Kleidungsstück nach einem der vorhergehenden Ansprüche, welches ferner zumindest an einem Endabschnitt der absorbierenden Anordnung eine Taillenelastik (44) aufweist.

18. Kleidungsstück nach Anspruch 17, wobei die Taillenelastik (44) eine Mehrzahl von elastischen Elementen aufweist.

19. Kleidungsstück gemäß Anspruch 1, wobei jedes der elastischen, dehnbaren Seitenbahnen (6, 8) einen Zwischenabschnitt (Z) aufweist, wobei der Zwischenabschnitt (Z) im allgemeinen peripher entlang eines Schrittbereichs einer jeweiligen Schenkelöffnung (12, 14) angeordnet ist, wobei die dehnbaren Seitenbahnen (6, 8) im allgemeinen nach innen gerichtete Kraftvektoren gegen einen Träger bilden, um das Kleidungsstück (2) enganliegend am Körper (16) des Trägers und die absorbierende Anordnung in enganliegender Position im Schrittbereich sowohl vor als auch nach einer Beaufschlagung zu halten.

20. Wegwerfbares Kleidungsstück gemäß Anspruch 19, wobei die elastischen dehnbaren Seitenbahnen (6, 8) zwei elastische dehnbare Seitenelemente (18, 20, 24, 26) aufweisen.

21. Kleidungsstück nach Anspruch 19 oder 20, wobei die Breite jedes Zwischenabschnitts zwischen 5% und 100% der maximalen Breite der jeweiligen der Seitenbahnen (6, 8) beträgt.

22. Kleidungsstück nach Anspruch 19, wobei jeder Zwischenabschnitt vorgedehnt ist, bevor er verbunden wird.

23. Kleidungsstück nach Anspruch 19, wobei die Innenseiten (98, 100) der elastischen dehnbaren Seitenelemente (24, 26) direkt an den jeweiligen Seiten (102, 104) des absorbierenden Mediums (92) anliegen.

24. Kleidungsstück nach Anspruch 19, wobei die Innenseiten (98, 100) der elastischen dehnbaren Seitenelemente (24, 26) etwa 0 cm bis etwa 5,08 cm (0 Inch bis etwa 2 Inch) von den jeweiligen Seiten (102, 104) des absorbierenden Mediums (92) beabstandet sind.

25. Kleidungsstück nach einem der Ansprüche 19 bis 24, wobei sich jede der Seitenbahnen (6, 8) über eine entsprechende Seite des absorbierenden Mediums (92) über eine Länge von 0% bis 50% der maximalen Breite des absorbierenden Mediums (92) erstreckt.

26. Kleidungsstück nach einem der Ansprüche 19 bis 25, wobei mindestens ein Saum (30, 32) manuell auftrennbar ist.

27. Kleidungsstück nach einem der Ansprüche 19 bis 26, wobei jede der Seitenbahnen (6, 8) einen Spannungsbereich von 50 bis 1000 g pro 2,54 cm (1 Inch) aufweist.

28. Kleidungsstück nach einem der Ansprüche 19 bis 27, welches ferner an jedem Endabschnitt der absorbierenden Anordnung eine Taillenelastik (42) aufweist.

29. Kleidungsstück nach Anspruch 28, wobei die Taillenelastik (42) ein aktivierbares elastisches Material ist.

30. Kleidungsstück nach einem der vorhergehenden Ansprüche, wobei jede der Seitenbahnen (6, 8) ein streckgebundener Schichtstoff (80) ist.

31. Kleidungsstück nach Anspruch 30, wobei der streckgebundene Schichtstoff (80) mindestens zwei Schichten (82, 84, 86) aufweist, wobei eine der Schichten (86) aus einem elastomeren Material hergestellt ist.

32. Kleidungsstück nach Anspruch 30, wobei der streckgebundene Schichtstoff (89) drei Schichten (82, 84, 86) aufweist, wobei die mittlere Schicht (86) aus einem elastomeren Material hergestellt ist.

33. Kleidungsstück nach einem der vorhergehenden Ansprüche, wobei die äußere Abdeckung (90) mindestens zwei Schichten aufweist, wobei die äußerste der Schichten aus einem tuchähnlichen Material und die innerste Schicht aus einem flüssigkeitsundurchlässigen Material hergestellt ist.

34. Kleidungsstück nach Anspruch 33, wobei die äußerste Schicht ein spinngebundenes thermoplastisches Material ist.

35. Kleidungsstück nach Anspruch 33, wobei die äußerste Schicht eine gebundene kardierte Bahn aus thermoplastischem Material ist.

36. Kleidungsstück nach Anspruch 33, wobei die innerste Schicht aus einem flüssigkeitsundurchlässigen schmelzgeblasenen thermoplastischen Material ist.

37. Kleidungsstück nach Anspruch 33, wobei die innerste Schicht ein extrudierter Film aus thermoplastischem Material ist.

38. Kleidungsstück nach einem der vorhergehenden Ansprüche, wobei jede der Seitenbahnen (6, 8) eine Elastizität von 10% bis 500% aufweist.

39. Kleidungsstück nach einem der Ansprüche 1 bis 19, mit einem Endsegmentabschnitt (136, 138) jeder der Seitenbahnen (6, 8), der winkelig im allgemeinen nach innen in Richtung der Taillenöffnung (10) angeordnet ist, um dadurch einen besseren Sitz an der Taille des Trägers (16) zu ermöglichen.

40. Kleidungsstück nach Anspruch 39, wobei die Länge jedes der Endsegmentabschnitte (136, 138) 3% bis 40%, vorzugsweise 5% bis 25%, und noch bevorzugter 10% bis 15%, der Gesamtlänge des Kleidungsstücks beträgt.

41. Kleidungsstück nach Anspruch 39 oder 40, wobei jeder Endsegmentabschnitt (136, 138) nach innen in Richtung der Taillenöffnung (10) in einem Winkel von 5° bis 55°, vorzugsweise 10° bis 40°, und noch bevorzugter 15° bis 30°, abgewinkelt ist.

42. Kleidungsstück nach einem der Ansprüche 38 bis 41, wobei der vordere Kantenbereich (132) einer jeden Schenkelöffnung (12, 14) näher an der Taillenöffnung (10) liegt als der hintere Kantenbereich (133).

43. Kleidungsstück nach einem der Ansprüche 38 bis 42, wobei die Seitenbahnen (6, 8) zwischen 20% und 80%, vorzugsweise 25% und 50%, und noch bevorzugter zwischen 35% und 45%, des gesamten Oberflächenbereichs des Kleidungsstücks (2) betragen.

44. Kleidungsstück nach einem der Ansprüche 38 bis 43, wobei die Breite eines jeden Zwischenabschnitts zwischen 5% und 100% der maximalen Breite der jeweiligen Seitenbahn (6, 8) beträgt.

45. Kleidungsstück gemäß einem der vorhergehenden Ansprüche, wobei mindestens ein Saum (30, 32) die elastischen dehnbaren Seitenbahnen (6, 8) verbindet.

46. Kleidungsstück gemäß Anspruch 1, wobei jede der Seitenbahnen (6, 8) zwei elastische dehnbare Seitenelemente (18, 20, 24, 26) aufweist, welche sich an ein Zwischenelement (22, 28) anschließen, welches aus einem nicht-dehnbaren Material hergestellt ist, wobei beide Zwischenelemente (22, 28) aus zwei Hälften hergestellt sind, welche durch jeweilige Säume (30, 32) verbunden sind, und wobei ein Zwischenabschnitt jeder dehnbaren Seitenbahn (6, 8) im allgemeinen peripher entlang eines Schrittbereichs einer jeweiligen der Schenkelöffnungen (12, 14) angeordnet ist, wobei die dehnbaren Seitenbahnen (6, 8) im allgemeinen nach innen gerichtete Kraftvektoren gegen einen Träger bilden, um das Kleidungsstück enganliegend am Körper (16) des Trägers und die absorbierende Anordnung in enganliegender Position im Schrittbereich sowohl vor als auch nach einer Beaufschlagung zu halten.

## Revendications

1. Vêtement jetable (2) analogue à une culotte, destiné à absorber un déversement humain, comprenant :
- un ensemble absorbant comprenant une enveloppe extérieure imperméable aux liquides (90), une doublure perméable aux liquides (88) et un milieu absorbant (92) ; ledit ensemble absorbant comprenant en outre des bords latéraux généralement opposés et des bords d'extrémité généralement opposés, et
- des moyens de fronçage (46) fournissant de l'élasticité le long d'au moins des portions (76,78) d'une paire d'ouvertures de jambes (12,14) dudit vêtement (2) pour éviter des fuites à ce niveau,
caractérisé par
- des panneaux latéraux extensibles élastiques (6,8) réunis auxdits bords latéraux pour former, avec ledit ensemble absorbant, une culotte d'apprentissage de la propreté pour enfants ayant une ouverture de taille (10) et lesdites ouvertures de jambes (12,14), grâce à quoi lesdits panneaux latéraux extensibles élastiques (6,8) maintiennent étroitement ladite culotte d'apprentissage de la propreté (2) contre le corps d'un porteur (16).

2. Vêtement selon la revendication 1, dans lequel lesdits moyens de fronçage ont une longueur fixée relâchée de 10% à 100%, de préférence de 10% à 50%, et mieux de 15% à 25%, de la longueur totale dudit vêtement.

3. Vêtement selon la revendication 1 ou 2, dans lequel lesdits moyens de fronçage (46) ont une élasticité de 25% à 350%, de préférence de 30% à 260%, et mieux de 125% à 200%.

4. Vêtement selon l'une quelconque des revendications 1 à 3, dans lequel chacun desdits moyens de fronçage (46) est constitué d'une pluralité d'éléments élastiques allongés.

5. Vêtement selon l'une quelconque des revendications 1 à 4, dans lequel chacun desdits moyens de fronçage (46) est pré-étiré lorsqu'il est réuni à son ouverture de jambe respective.

6. Vêtement selon l'une quelconque des revendications 1 à 4, dans lequel chacun desdits moyens de fronçage (46) est un prolongement d'un seul tenant de l'un desdits panneaux latéraux extensibles élastiques (6,8).

7. Vêtement selon la revendication 6, dans lequel chacun desdits prolongements d'un seul tenant est pré-étiré lorsqu'il est réuni audit ensemble absorbant.

8. Vêtement selon l'une quelconque des revendications 1 à 7, qui comprend en outre une paire de sections latérales sensiblement non extensibles (22,28), chacune desdites sections latérales étant réunie en une position intermédiaire d'un panneau latéral extensible élastique (6,8) respectif.

9. Vêtement selon l'une quelconque des revendications précédentes, dans lequel chacun desdits panneaux latéraux extensibles élastiques (6,8) comprend deux éléments latéraux extensibles élastiques (18,20,22,24).

10. Vêtement selon les revendications 8 et 9, dans lequel lesdites sections latérales (22,28) sont réunies par des jonctions (34,36,38,40) aux éléments latéraux extensibles (18,20,24,26).

11. Vêtement selon l'une quelconque des revendications précédentes, dans lequel l'un au moins desdits panneaux latéraux extensibles élastiques (6,8) est déchirable manuellement depuis ledit ensemble absorbant.

12. Vêtement selon l'une quelconque des revendications précédentes, dans lequel les portions marginales desdits moyens de fronçage (46) viennent directement en butée contre les côtés respectifs dudit milieu absorbant (92).

13. Vêtement selon l'une quelconque des revendications précédentes, dans lequel les portions marginales desdits moyens de fronçage (46) sont écartées des côtés respectifs dudit milieu absorbant d'une distance de 0 cm à 5,08 cm (0 pouce a 2 pouces), de préférence de 0 cm à 2,54 cm (0 pouce à 1 pouce), et mieux de 0 cm à 1,27 cm (0 pouce à 1/2 pouce).

14. Vêtement selon l'une quelconque des revendications précédentes, dans lequel chacune des portions d'extrémité desdits moyens de fronçage (46) s'étend sur un côté respectif dudit milieu absorbant sur une distance de 0% à 50%, de préférence de 3% à 20%, et mieux de 6% à 12%, de la largeur maximale dudit milieu absorbant.

15. Vêtement selon l'une quelconque des revendications précédentes, dans lequel chacun desdits panneaux latéraux extensibles élastiques (6,8) a une gamme de tensions pour 2,54 cm (1 pouce) comprise entre 50 grammes et 1000 grammes, et de préférence 200 grammes et 500 grammes.

16. Vêtement selon l'une quelconque des revendications précédentes, dans lequel la largeur de chacun desdits moyens de fronçage (46) est comprise entre 1% et 100%, de préférence 20% et 80%, et mieux 30% et 50%, de la largeur maximale de son panneau latéral respectif (6,8).

17. Vêtement selon l'une quelconque des revendications précédentes, comprenant en outre un élastique de taille (44) au moins à une portion d'extrémité dudit ensemble absorbant.

18. Vêtement selon la revendication 17, dans lequel ledit élastique de taille (44) est constitué d'une pluralité d'éléments élastiques.

19. Vêtement selon la revendication 1, dans lequel chacun desdits panneaux latéraux extensibles élastiques (6,8) comprend une portion intermédiaire (Z), ladite portion intermédiaire (Z) étant généralement disposée périphériquement le long d'une portion d'entrejambe d'une ouverture de jambe (12,14) respective, de sorte que lesdits panneaux latéraux extensibles (6,8) fournissent des vecteurs de force, généralement dirigés vers l'intérieur, contre un porteur pour maintenir étroitement ledit vêtement (2) contre le corps du porteur (16) et ledit ensemble absorbant étroitement en place contre la zone d'entrejambe, à la fois avant et après un déversement.

20. Vêtement jetable selon la revendication 19, dans lequel lesdits panneaux latéraux extensibles élastiques (6,8) comprennent des éléments latéraux extensibles (18,20,24,26).

21. Vêtement selon la revendication 19 ou 20, dans lequel la largeur de chacune desdites portions intermédiaires est comprise entre 5% et 100% de la largeur maximale de son panneau latéral respectif (6,8).

22. Vêtement selon la revendication 19, dans lequel chacune desdites portions intermédiaires est pré-étirée avant d'être réunie.

23. Vêtement selon la revendication 19, dans lequel les côtés intérieurs (98,100) des éléments latéraux extensibles élastiques (24,26) viennent directement en butée contre les côtés respectifs (102,104) dudit milieu absorbant (92).

24. Vêtement selon la revendication 19, dans lequel les côtés intérieurs (98,100) des éléments latéraux extensibles élastiques (24,26) sont écartés des côtés respectifs (102,104) dudit milieu absorbant (92) d'une distance d'environ 0 cm à environ 5,08 cm (0 pouce à environ 2 pouces).

25. Vêtement selon l'une quelconque des revendications 19 à 24, dans lequel chacun desdits panneaux latéraux (6,8) s'étend sur un côté respectif dudit milieu absorbant (92) sur une distance de 0% à 50% de la largeur maximale dudit milieu absorbant (92).

26. Vêtement selon l'une quelconque des revendications 19 à 25, dans lequel au moins une jonction (30,32) est déchirable manuellement.

27. Vêtement selon l'une quelconque des revendications 19 à 26, dans lequel chacun desdits panneaux latéraux (6,8) a une gamme de tensions pour 2,54 cm (1 pouce) comprise entre 50 grammes et 1000 grammes.

28. Vêtement selon l'une quelconque des revendications 19 à 27, comprenant en outre un élastique de taille (42) à chaque portion d'extrémité dudit ensemble absorbant.

29. Vêtement selon la revendication 28, dans lequel ledit élastique de taille (42) est formé d'un matériau élastique activable.

30. Vêtement selon l'une quelconque des revendications précédentes, dans lequel chacun desdits panneaux latéraux (6,8) est un stratifié lié à l'état étiré (80).

31. Vêtement selon la revendication 30, dans lequel ledit stratifié lié à l'état étiré (80) est formé d'au moins deux couches (82,84,86), l'une desdites couches (86) étant faite d'un matériau élastomère.

32. Vêtement selon la revendication 30, dans lequel ledit stratifié lié à l'état étiré (80) est formé de trois couches (82,84,86), la couche intermédiaire (86) étant faite d'un matériau élastomère.

33. Vêtement selon l'une quelconque des revendications précédentes, dans lequel ladite enveloppe extérieure (90) est formée d'au moins deux couches, la couche la plus extérieure desdites couches étant faite d'un matériau semblable à un textile et la couche la plus intérieure desdites couches étant faite d'un matériau imperméable aux liquides.

34. Vêtement selon la revendication 33, dans lequel la couche la plus extérieure est faite d'un matériau thermoplastique lié au filage.

35. Vêtement selon la revendication 33, dans lequel la couche la plus extérieure est faite d'une nappe cardée liée de matériau thermoplastique.

36. Vêtement selon la revendication 33, dans lequel la couche la plus intérieure est faite d'un matériau thermoplastique obtenu par fusion-soufflage, imperméable aux liquides.

37. Vêtement selon la revendication 33, dans lequel la couche la plus intérieure est faite d'un film extrudé de matériau thermoplastique.

38. Vêtement selon l'une quelconque des revendications précédentes, dans lequel chacun desdits panneaux latéraux (6,8) a une élasticité comprise entre 10% et 500%.

39. Vêtement selon l'une quelconque des revendications 1 à 19, comprenant une portion de segment d'extrémité (136,138) de chacun desdits panneaux latéraux (6,8) qui est disposée en faisant un angle généralement vers l'intérieur en direction de ladite ouverture de taille (10), de façon à réaliser un meilleur ajustage à la taille du porteur (16).

40. Vêtement selon la revendication 39, dans lequel la longueur de chacune desdites portions de segment d'extrémité (136,138) est comprise entre 3% et 40%, de préférence 5% et 25%, et mieux 10% et 15%, de la longueur totale dudit vêtement.

41. Vêtement selon la revendication 39 ou 40, dans lequel chacune desdites portions de segment d'extrémité (136,138) fait un angle vers l'intérieur en direction de ladite ouverture de taille (10), ledit angle étant compris entre 5° et 55°, de préférence 10° et 40°, et mieux 15° et 30°.

42. Vêtement selon l'une des revendications 38 à 41, dans lequel la portion de bord avant (132) de chacune desdites ouvertures de jambes (12,14) est plus proche de ladite ouverture de taille (10) que ne l'est la portion de bord arrière (133).

43. Vêtement selon l'une des revendications 38 à 42, dans lequel lesdits panneaux latéraux (6,8) représentent d'environ 20% à 80%, de préférence 25% à 50%, et mieux 35% à 45%, de l'aire superficielle totale dudit vêtement (2).

44. Vêtement selon l'une des revendications 38 à 43, dans lequel la largeur de chacune desdites portions intermédiaires est comprise entre 5% et 100% de la largeur maximale de son panneau latéral respectif (6,8).

45. Vêtement selon l'une des revendications précédentes, dans lequel au moins une jonction (30,32) réunit lesdits panneaux extensibles élastiques (6,8).

46. Vêtement selon la revendication 1, dans lequel chacun desdits panneaux latéraux (6,8) comprend deux éléments latéraux extensibles élastiques (18,20,24,26) connectant un élément intermédiaire (22,28) qui est constitué d'un matériau non extensible, les deux éléments intermédiaires (22,28) étant constitués de deux moitiés réunies par des jonctions (30,32) respectives et une portion intermédiaire de chaque panneau latéral extensible (6,8) étant disposée généralement périphériquement le long d'une portion d'entrejambe d'une ouverture de jambe (12,14) respective, grâce à quoi lesdits panneaux latéraux extensibles (6,8) créent des vecteurs de force, dirigés généralement vers l'intérieur, contre un porteur pour maintenir étroitement ledit vêtement contre le corps du porteur (16) et ledit ensemble absorbant étroitement en place contre la zone d'entrejambe, à la fois avant et après un déversement.
